# EUROPÄISCHE PATENTSCHRIFT

(11) **EP 1 027 377 B1**
(45) Veröffentlichungstag und Bekanntmachung des Hinweises auf die Patenterteilung: **23.04.2003**
(21) Anmeldenummer: 98955546.1
(22) Anmeldetag: 06.11.1998
(51) Int. Cl.: C08F 8/14, C09D 4/06, C09J 4/06, A61K 6/00

(54) **POLYMERISIERBARE ZUSAMMENSETZUNG UND IHRE VERWENDUNG ALS HAFTVERMITTLER**
POLYMERIZABLE COMPOSITION AND THE APPLICATION THEREOF AS A COUPLING AGENT
COMPOSITION POLYMERISABLE ET SON UTILISATION COMME AGENT ADHESIF

(30) Priorität: 07.11.1997 DE 19749349
(43) Veröffentlichungstag der Anmeldung: 16.08.2000
(73) Patentinhaber: 3M ESPE AG, 82229 Seefeld (DE)
(72) Erfinder: MAY, Robert, D-82229 Seefeld (DE); MIKULLA, Markus, D-82346 Andechs-Frieding (DE); BISSINGER, Peter, D-86415 Mering (DE)
(74) Vertreter: Freiherr von Wittgenstein, Arved, Dr.
(86) Internationale Anmeldenummer: EP9807092
(87) Internationale Veröffentlichungsnummer: WO99024477

(56) Entgegenhaltungen:
- EP-A- 0 325 038
- DE-A- 2 635 123
- US-A- 5 658 963

## Beschreibung

Die Erfindung betrifft polymerisierbare, vernetzbare Zusammensetzungen, enthaltend Polyalkylbenzolderivate und/oder Derivate ringförmiger Polycarbonsäuren und/oder deren Anhydride sowie deren Verwendung, insbesondere auf dem dentalen Bereich. Derartige Zusammensetzungen haften auf verschiedenen, besonders aber auf biologischen Substraten. Verbindungen dieser Art können für allgemeine Zwecke, aber besonders im dentalen Bereich als Komponenten für polymerisierbare Haftvermittler, Haftfüllungsmaterialien, Haftzemente, Sealer und für ähnliche Mischungen eingesetzt oder zugesetzt werden.

Polymerisierbare Mischungen auf der Basis von monomeren, mit einer oder mehreren ungesättigten Gruppen versehenen Verbindungen bilden die Grundlage für eine Vielzahl von Kunststoffen, wobei im dentalen und medizinischen Bereich hauptsächlich Verbindungen mit Acrylat- und Methacrylatgruppen wichtig sind. Mischungen dieser Art sind z.B. die Grundlage für Kunststofffüllungs- und Versiegelungsmaterialien. Diese polymerisierbaren Mischungen können jedoch im allgemeinen keine chemische Verbindung mit anderen Materialien, besonders biologischen Substraten, ausbilden, es sei denn, diese Substrate enthalten noch selbst ausreichend unpolymerisierte polymerisierbare Gruppen.

Eine festere Verbindung kann daher nur über retentionsreiche Flächen, d.h. über einen Verbund rein mechanischer Art, z.B. nach Anätzen der Oberfläche von biologischen oder anorganischen Materialien erreicht werden. Durch Anwendung von Haftvermittlern, d.h. von Substraten, die einerseits mit biologischem oder anorganischem Material chemisch reagieren können als auch andererseits eine polymerisierbare Gruppe tragen, kann dieser Mangel jedoch behoben werden.

Es ist eine Reihe von solchen Haftvermittlern bekannt, z.B. die Vinyl- oder Methacrylgruppen tragenden Organosilane. Sie sind jedoch in ihrer Haftwirkung auf Siliciumdioxid, Siliciumdioxid enthaltende Gläser sowie Keramiken und Metalloxide, bzw. diese bildende Unedelmetalle beschränkt. Auf biologischen Substraten und besonders auf Zahn- oder Knochensubstrat zeigen sie keine Haftung, sondern wirken eher trennend. Für Substrate solcher Art sind eine Reihe von polymerisierbaren Haftvermittlern mit anderen Haftgruppen gefunden worden. Es gibt solche, die mit dem Collagen oder collagenähnlichen Anteilen dieser Substrate reagieren, wie z.B.
- 2-N-Allylamino-4,6-dichlor-1,3,5-triazin (US-A-4 203 220),
- Kombinationen von Hydroxy-Methacrylester mit Dialdehyden (EP-A-0141324) oder
- Epoxymethacrylate.

Ferner gibt es eine Zahl von polymerisierbaren Verbindungen, die mit den Apatit-Verbindungen der Zahn- oder Knochensubstanz reagieren. Diese haftvermittelnden Verbindungen tragen Säuregruppen oder reaktive Säuregruppenderivate. Beispiele solcher polymerisierbare Verbindungen sind:
- ungesättigte organische Ester von Phosphor- oder Phosphonsäuren (DE-A-27 11 234, DE-A-31 50 285);
- ungesättigte organische Ester der Monofluorphosphorsäure (US-A-3 997 504);
- ungesättigte organische Ester von Säuren des Phosphors, die Chlor oder Brom direkt am Phosphor gebunden enthalten (EP-A-0 058 483);
- ungesättigte organische Ester der Phosphorsäure, die als cyclische Pyrophosphate (Anhydride) vorliegen (DE-A-30 48 410).

Es sind auch polymerisierbare Carbonsäuren und reaktive Carbonsäurederivate bekannt, die Haftung zur Zahnsubstanz zeigen, wie z.B.:
- 4-Methacryloyloxyethyltrimellitsäure und deren Anhydrid (Takeyama, M. et al., J. Jap. Soc. f. Dent. App. a. Mat. 19, 179 (1978)) oder
- Methacroyloxy-ethyl-o-phthalat (E. Mashuhara, K. Kojhima, N. Tarumi, N. Nakabayashi, H. Hotta, Rep. Inst. Med. Dent. Eng. 1, 29, (1967).

Auch Reaktionsprodukte von ethylenisch ungesättigten Alkoholen mit 3 bis 12 C-Atomen mit cyclischen Anhydriden sind als Haftvermittler im Einsatz (US-A-4 659 751, Bowen).

Systeme auf der Basis von Phosphorsäure sind äußerst hydrolyseempfindlich und solche auf der Basis von Carbonsäuren mit dem Nachteil behaftet, daß eine Bindung mit dem Substrat nicht optimal vollzogen werden kann. Die meisten Haftmoleküle sind außerdem synthetisch schwer zugänglich und sind daher nur schwer wirtschaftlich zu nutzen. Es besteht somit ein hohes Interesse, leicht zugängliche Haftmoleküle zu erhalten, die hydrolysestabil, leichter zur Substratbindung befähigt als die Moleküle aus dem Stand der Technik sowie kostengünstig und wirtschaftlich herstellbar sind.

Der Erfindung liegt die Aufgabe zugrunde, polymerisierbare und vernetzbare Zusammensetzungen zur Verfügung zu stellen, die eine hohe Haftung, insbesondere an biologischen Substraten und ganz besonders an der Zahnhartsubstanz und am Dentin, zeigen.

Überraschenderweise zeigen Zusammensetzungen, die Moleküle enthalten, welche aus der Umsetzung von Molekülen der allgemeinen Formel (1) oder von ringförmigen Polycarbonsäuren und/oder deren Anhydriden mit gesättigtem Ringsystem und mindestens vier Carboxylgruppen sowie 0 oder 1 Heteroringatomen aus der Gruppe N, O, S mit OH-funktionellen (Meth)acrylaten entstanden sind (allgemeine Formel (3)), eine über das bekannte Maß hinausgehende Haftung an biologischen Substraten, insbesondere an der Zahnhartsubstanz wie Schmelz und Dentin. Durch die Kombination dieser Moleküle in einer haftvermittelnden Zusammensetzung wird unerwarteterweise eine weitere Erhöhung der Haftung beobachtet.

Die Erfindung wird nachstehend im Einzelnen beschrieben.

Unter (Meth)acrylaten sind im folgenden Methacrylsäureester, Acrylsäureester, Methacrylamide, Acrylamide sowie Thiomethacrylsäureester und Thioacrylsäureester zu verstehen.

Die erfindungsgemäßen polymerisierbaren, vernetzbaren Zusammensetzungen müssen neben anderen Komponenten, wie beispielsweise Füllstoffe, Monomere oder Lösungsmittel, haftvermittelnde Substanzen enthalten.

Die erfindungsgemäße Zusammensetzung besteht aus:
(A) 1 bis 99,99 Gew.-%, bevorzugt 5 bis 70 Gew.-% von Umsetzungsprodukten von OH-funktionellen (Meth)acrylaten mit
   (i) den Verbindungen der Formel (1): 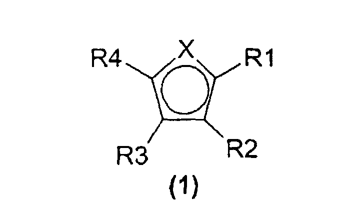 in welcher:
      R1, R2, R3 und R4 unabhängig voneinander ausgewählt sind aus der Gruppe:
         (a) Wasserstoff,
         (b) unverzweigte oder verzweigte Kohlenwasserstoffreste mit 1 bis 20 C-Atomen, bevorzugt Methyl,
         (c) unverzweigte oder verzweigte Heteroaliphaten mit 1 bis 20 C-Atomen und Heteroatomen ausgewählt aus der Gruppe N, O, S, bevorzugt O,
         (d) Halogene und Pseudohalogene, bevorzugt F, Cl, Br, CN, SCN, ganz besonders bevorzugt Cl, Br und
         (e) dem Fragment der Formel (2): 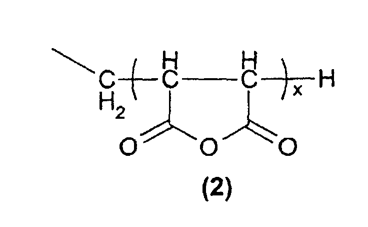 in welcher x ganze Zahlenwerte zwischen 1 und 8 annehmen kann, und X ausgewählt ist aus der Gruppe O, S, 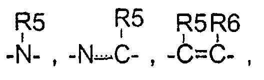 bevorzugt 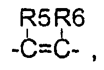 mit R5 bzw. R6 entsprechend der obigen Definition von R1 bis R4, mit der Maßgabe, daß mindestens zwei beliebige Vertreter der Gruppe R1 bis R4, R5, R6 dem Fragment der Formel (2) entsprechen, wobei das Substitutionsmuster der Fragmente der Formel (2) ortho, meta oder para sein kann (Umsetzungsproduktgruppe A1) und wobei die Kombination R1 bis R4 = H, 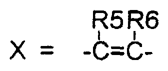 mit R5 = R6 = Fragment der Formel (2) bevorzugt ist,
      und/oder mit
   (ii) ringförmigen Polycarbonsäuren mit mindestens 4 Ring-Kohlenstoffatomen und/oder deren Anhydriden mit gesättigtem Ringsystem und mind. vier Carboxylgruppen sowie 0 oder 1 Hetero-Ringatomen aus der Gruppe N, O, S (Umsetzungsproduktgruppe A2) der allgemeinen Formel (3), 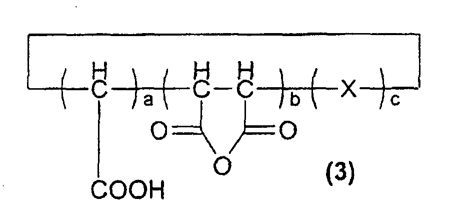
   worin a = 0 bis 6,
   b = 0 bis 3,
   c = 0 oder 1, und
   X = O, NR1 oder S bedeuten,
   wobei R1 die obige Bedeutung hat, ausgenommen (e) ein Fragment der Formel (2), und
   wobei mindestens eine Carboxylgruppe und/oder Anhydridgruppe der vorstehend bezeichneten Substanzgruppen mit ungesättigten polymerisierbaren Gruppen funktionalisiert ist,
(B) 0 bis 98,99 Gew.-%, bevorzugt 5 bis 70 Gew.-% einer oder mehrerer polymerisierbarer, ungesättigter organischer Verbindungen mit wenigstens einer CH₂=C(R10)-COO-Gruppe, worin R10 = H oder Methyl ist;
(C) 0,01 bis 5 Gew.-%, bevorzugt 0,01 bis 3 Gew.-% Initiatoren und ggf. Aktivatoren;
(D) 0 bis 90 Gew.-%, bevorzugt 0 bis 50 Gew.-% übliche Lösungsmittel;
(E) 0 bis 90 Gew.-%, bevorzugt 20 bis 80 Gew.-% übliche Füllstoffe, Pigmente, Thixotropiehilfsmittel, Weichmacher, Verdünner, starrmachende Monomere, Radikalfänger, Stabilisatoren, sonstige Hilfsmittel,
wobei sich die Angabe der Gew.-% jeweils auf die Summe aller Bestandteile (A) bis (E) bezieht.

Bevorzugte Verbindungen der Formel (1) entsprechen der folgenden Formel (4) 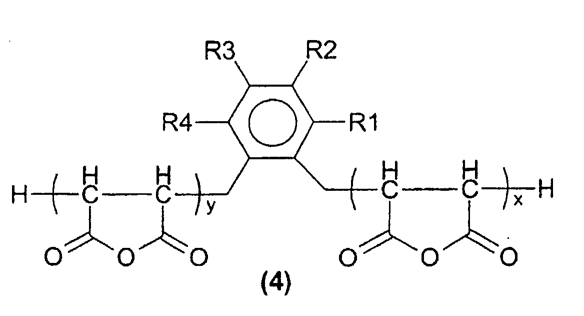 in welcher x und y unabhängig voneinander ganze Zahlenwerte zwischen 1 und 8 annehmen können und R1, R2, R3 und R4 die oben angegebenen Bedeutungen gemäß Formel (1) (a) - (d) haben. Bevorzugt sind weiterhin die entsprechenden Verbindungen, bei denen die die Anhydridgruppen tragenden Substituenten nicht in ortho-, sondern in meta- oder in para-Stellung am Benzolring angeordnet sind.

Die Synthese von Verbindungen der allgemeinen Formel (1) ist bekannt. Die DE-A-2 405 284 (Ciba-Geigy), EP-A-0 383 724 (Ciba-Geigy), GB-A-1 529 092 (Ciba-Geigy) und GB-A-2 164 339 beschreiben die Polymerisation von Maleinsäureanhydrid unter Anwendung eines Peroxidinitiators in einer verdünnten Lösung in o-Xylol. Die Synthese kann in der Weise durchgeführt werden, daß die drei Ausgangssubstanzen Xylylenverbindung, bevorzugt 1,2-Xylol, Maleinsäureanhydrid und Radikalinitiator, beispielsweise Di-tert.-butylperoxid, tert.-Butylhydroperoxid, Dibenzoylperoxid oder α,α'-Azo-isobutyronitril zusammengegeben und erhitzt werden. Der Initiator kann auch zum Gemisch der beiden anderen Edukte dosiert werden. Je nach Einsatzstoffen und Reaktionstemperatur kann die Reaktion wenige Stunden bis mehrere Tage benötigen. Nach beendeter Reaktion wird das Produkt durch Entfernen der überschüssigen Xylylenkomponente als Rohsubstanz erhalten und kann ohne weitere Reinigung für den nächsten Syntheseschritt eingesetzt werden.

Das Produkt dieser ersten Synthesestufe enthält gemäß der allgemeinen Formeln (1) und (2) ein aus der verwendeten Xylylenkomponente hervorgegangenes Struktursegment, das über Methylengruppen kovalent mit je einem einzelnen Maleinsäureanhydridfragment (MSA-Fragment) oder mehreren miteinander kovalent verknüpften MSA-Fragmenten verbunden ist. Demgemäß enthält das Produkt ein Gemisch aus Substanzen der allgemeinen Formeln (1) und (2) mit verschieden hoher Anhydridfunktionalität. Bei Einsatz von 1,2-Xylol entsteht bevorzugt ein Produkt mit x = 4 bis 6 MSA-Fragmenten. Wird 1,4-Xylol oder Mesitylen verwendet, liegt die Zahl der angeknüpften MSA-Einheiten x bevorzugt bei 2 bis 3.

In einem zweiten Syntheseschritt wird das Reaktionsprodukt der allgemeinen Formeln (1) und (2) mit einem OH-funktionellen (Meth)acrylat, bevorzugt 2-Hydroxyethylmethacrylat (HEMA), 3-Hydroxypropyl(meth)acrylat, 2-Hydroxyethyl(meth)acrylamid, 2-Hydroxy-ethylthiol(meth)acrylat oder Isopropylidenbis-[2-hydroxy-3-(4-phenoxy)-propyl]-methacrylat, ganz besonders bevorzugt 2-Hydroxyethylmethacrylat partiell oder vollständig verestert (Umsetzungsproduktgruppe A1).

Dazu wird das Produkt der allgemeinen Formeln (1) und (2) in einem inerten organischen Solvens, beispielsweise Tetrahydrofuran (THF) gelöst und in Gegenwart eines sauren oder basischen Katalysators mit der gewünschten Menge OH-funktionellem (Meth)acrylat vollständig umgesetzt. Als Katalysatoren können beispielsweise Schwefelsäure, 4-Toluolsulfonsäure, Methansulfonsäure, Natriumacetat oder Pyridin eingesetzt werden. Die Reaktion wird bei Temperaturen zwischen 20 und 70°C, bevorzugt bei 50 bis 60°C durchgeführt. Je nach Stöchiometrie, Anhydrid/OH-funktionelles (Meth)acrylat, Temperatur und Katalysator sind Reaktionszeiten von einem bis sieben Tagen erforderlich.

Das verwendete Verhältnis Anhydrid/OH-funktionelles (Meth)acrylat ist bevorzugt kleiner als eins, besonders bevorzugt 0,1 bis 0,9 und ganz besonders bevorzugt 0,2 bis 0,7.

Die Vollständigkeit der Reaktion kann mittels Dünnschichtchromatographie oder NMR-Spektroskopie überprüft werden. Wird die Stöchiometrie Anhydrid/OHfunktionellem (Meth)acrylat größer als eins gewählt, kann das nach der Reaktion verbliebene Anhydrid mit Wasser hydrolysiert werden. Diese Reaktion läßt sich mittels IR-Spektroskopie verfolgen.

Das Endprodukt wird nach Extraktion des Ansatzes mittels eines organischen Lösungsmittels, wie z.B. Diethylether, Methyl-tert.-butylether, Chloroform, Methylenchlorid oder Toluol erhalten. Hierbei sind die Fragmente der Formel (2) zu Fragmenten der Formel (5) umgesetzt worden: 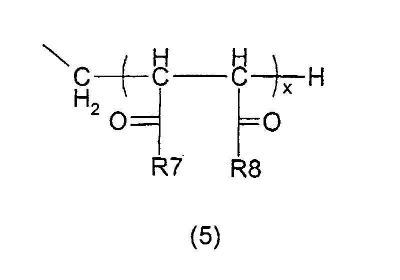 wobei die Definition von x derjenigen in der allgemeinen Formel (2) entspricht und R7 und R8 unabhängig voneinander OH, OR9 sein können, wobei mindestens ein Vertreter der Gruppe R7 und R8 OR9 sein muß, wobei R9 eine (Meth)acrylatfunktionalisierte Gruppe bedeutet, vorzugsweise eine aliphatische, aromatische oder araliphatische Gruppe, wobei die aliphatischen Gruppen vorzugsweise 4 bis 10 G-Atome enthalten, beispielsweise 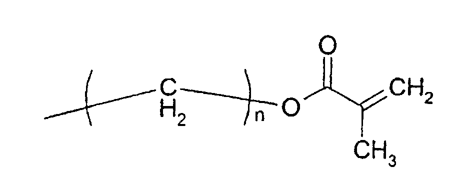 mit n = 1 bis 7, vorzugsweise n = 2 oder 3
oder

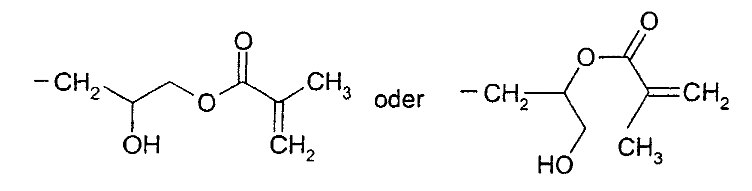

Das Substitutionsmuster der beiden Fragmente der Formeln (2) bzw. (5) kann ortho, meta oder para, bevorzugt ortho sein.

Auch Produkte, die durch eine unvollständige Umsetzung der Verbindungen der allgemeinen Formeln (1) und (2) über die vorgehend beschriebene Reaktion entstanden sind, also nicht umgesetzte Anhydridgruppen enthalten, gehören zum Umfang dieser Erfindung.

Das Endprodukt dieser Reaktion stellt ein Gemisch mehrerer Substanzen dar, von denen jede unterschiedlich viele Carbonsäure- und (meth)acrylatfunktionelle Estergruppen aufweisen kann. Demgemäß liegt kein einheitliches Molekulargewicht vor; das mittlere Molekulargewicht der Monomeren [(1) + (2)] liegt bei 300 bis 4000 g/mol, bevorzugt bei 600 bis 1000 g/mol. Zusätzlich sind die im Gemisch enthaltenen Verbindungen zueinander regioisomer, da bei der Reaktion des Produktes der allgemeinen Formeln (1) und (2) mit OH-funktionellem (Meth)acrylat die Anknüpfungen der OH-Gruppen an verschiedenen Anhydridgruppen und bei gegebener Anhydridgruppe an zwei verschiedenen C-Atomen stattfinden kann.

Die Ausgangsverbindungen der einen Komponente der Umsetzungsproduktgruppe A2 der beanspruchten Zusammensetzungen, nämlich die ringförmigen Polycarbonsäuren der allgemeinen Formel (3) mit mindestens 4 Ring-Kohlenstoffatomen mit gesättigtem Ringsystem sowie deren Anhydride mit mindestens 4, bevorzugt 4 bis 9, besonders bevorzugt 4 bis 7, ganz besonders bevorzugt 4 bis 6 Carboxylgruppen sowie 0 oder 1 Heteroringatomen aus der Gruppe N, O, S, bevorzugt N und O, besonders bevorzugt O, sind frei zugänglich.

Bevorzugt werden 2,3,4,5-Tetrahydrofurantetracarbonsäure und 1,2,3,4,5,6-Cyclohexanhexacarbonsäure sowie deren Anhydride eingesetzt. Diese Verbindungen sind frei verfügbar.

Die Umsetzung zum erfindungsgemäßen haftvermittelnden Baustein (Umsetzungsproduktgruppe A2) erfolgt durch Reaktion von OH-funktionellem (Meth)acrylat mit der Ausgangsverbindung in einem üblichen Lösungsmittel, beispielsweise Tetrahydrofuran. Bevorzugte OH-funktionelle (Meth)acrylate sind hierbei 2-Hydroxyethyl(meth)acrylat, 3-Hydroxypropyl(meth)acrylat, 2-Hydroxyethyl(meth)acrylamid, 2-Hydroxyethylthiol(meth)acrylat und Isopropylidenbis-[2-hydroxy-3-(4-phenoxy)-propyl]methacrylat. Als Produkt kann auch ein Gemisch aus teilweise veresterten Molekülen entstehen. Um den Veresterungsgrad zu steuern, können auch entsprechende Anhydride, die gleichfalls frei verfügbar sind, Einsatz finden. Im Falle des Dianhydrids von Tetrahydrofurantetracarbonsäure wird beispielsweise selektiv ein Diester erhalten.

Die Umsetzungsprodukte folgen demnach der allgemeinen Formel (6): 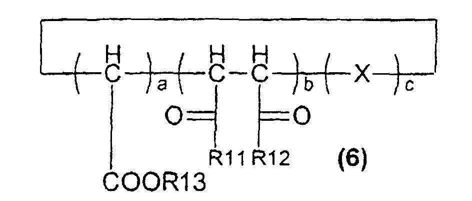 worin X, a, b und c wie oben definiert sind und R11, R12 und R13 unabhängig voneinander OH oder OR9 bedeuten können mit der Maßgabe, daß mindestens ein Vertreter der Gruppe R11, R12 und R13 OR9 sein muß, wobei R9 die obige Bedeutung hat. Für den Fall, daß b = 0 ist, gilt, daß mindestens eine Carbonsäuregruppe umgesetzt sein muß.

Der Bestandteil (A) ist in den erfindungsgemäßen Zusammensetzungen in einer Menge von 1 bis 99,99 Gew.-%, vorzugsweise 5 bis 70 Gew.-%, bezogen auf das Gesamtgewicht der Zusammensetzung, vorhanden.

Unter Bestandteil (B) der erfindungsgemäßen Zusammensetzungen, einer polymerisierbaren ungesättigten organischen Verbindung mit wenigstens einer CH₂=C(R10)-COO-Gruppe, wobei R10 = H oder Methyl, ist eine polymerisierbare ungesättigte organische Verbindung mit einer Acryloyl- oder Methyacryloylgruppe zu verstehen. Bevorzugt werden unter anderem Ester von Acryl- oder Methacrylsäure. Beispiele sind Methylmethacrylat, Methylacrylat, Ethylmethacrylat, Ethylacrylat, Propylmethacrylat, Propylacrylat, Isopropylmethacrylat, Isopropylacrylat, Hydroxymethylmethacrylat, Hydroxymethylacrylat, Hydroxyethylmethacrylat, Hydroxyethylacrylat, Hydroxypropylmethacrylat, Hydroxypropylacrylat, Tetrahydrofurfurylmethacrylat, Tetrahydrofurfurylacrylat, Glycidylmethacrylat, Glycidylacrylat, Triethylenglykoldimethacrylat, Triethylenglykoldiacrylat, Tetraethylenglykoldimethacrylat, Tetraethylenglykoldiacrylat, Trimethylolethantrimethacrylat, Trimethylolpropantrimethacrylat, Pentaerythritoltrimethacrylat, Trimethylolethantriacryrlat, Trimethylolpropantriacrylat, Pentaerythrittriacrylat, Pentaerythrittetramethacrylat, Pentaerythrittetraacrylat, Ethylendimethacrylat, Ethylendiacrylat, Ethylenglykoldimethacrylat, Ethylenglykoldiacrylat, Butylenglykoldimethacrylat, Butylenglykoldiacrylat, Neopentylglykoldimethacrylat, Neopentylglykoldiacrylat, 1,3-Butandioldimethacrylat, 1,3-Butandioldiacrylat, 1,4-Butandioldimethacrylat, 1,4-Butandioldiacrylat, 1,6-Hexandioldimethacrylat, 1,6-Hexandioldiacrylat, di-2-Methacryloxyethylhexamethylendicarbamat, Di-2-methacryloxyethyltrimethylhexamethylendicarbamat, Di-2-methacryloxyethyldimethylbenzoldicarbamat, Di-2-methacryloxyethyldimethylcyclohexandicarbamat, Methylenbis-2-methacryloxyethyl-4-cyclohexylcarbamat, Di-1-methyl-2-methacryloxyethylhexamethylendicarbamat, Di-1-methyl-2-methacryloxyethyltrimethylhexamethylendicarbamat, Di-1-methyl-2-methacryloxyethyldimethylbenzoldicarbamat, Di-1-methyl-2-methacryloxyethyldimethylcyclohexandicarbamat, Methylen-bis-1-methyl-2-methacryloxyethyl-4-cyclohexylcarbamat, Di-1-chlormethyl-2-methacryloxyethylhexamethylendicarbamat, Di-1-chlormethyl-2-methacryloxyethyltrimethylhexamethylendicarbamat, Di-1-chlormethyl-2-methacryloxyethyldimethylbenzoldicarbamat, Di-1-chlormethyl-2-methacryloxyethyldimethylcyclohexandicarbamat, Methylen-bis-2-methacryloxyethyl-4-cyclohexylcarbamat, Di-1-methyl-2-methacryloxyethylhexamethylendicarbamat, Di-1-methyl-2-methacryloxyethyltrimethylhexamethylendicarbamat, Di-1-methyl-2-methacryloxyethyldimethylbenzoldicarbamat, Di-1-methyl-2-methacryloxyethyldimethylcyclohexandicarbamat, Methylen-bis-1-methyl-2-methacryloxyethyl-4-cyclohexylcarbamat, Di-1-chlormethyl-2-methacryloxyethylhexamethlendicarbamat, Di-1-chlormethyl-2-methacryloxyethyltrimethylhexamethylendicarbamat, Di-1-chlormethyl-2-methacryloxyethyldimethylbenzodicarbamat, Di-1-chlormethyl-2-methacryloxyethldimethylcyclohexandicarbamat, Methylen-bis-1-chlormethyl-2-methacryloxyethyl-4-cyclohexylcarbamat, 2,2'-Bis(4-methacryloxyphenyl)-propan, 2,2'-Bis(4-acryloxyphenyl)propan, 2,2'-Bis-(4(2-hydroxy-3-methacryloxyphenyl))-propan, 2,2'-Bis(4(2-hydroxy-3-acryloxyphenyl))-propan, 2,2'-Bis(4-methacryloxyethoxyphenyl)-propan, 2,2'-Bis(4-acryloxyethoxyphenyl)-propan, 2,2'- Bis(4-ethacryloxypropoxyphenyl)-propan, 2,2'-Bis(4-acryloxydiethoxyphenyl)-propan, 2,2'-Bis(4-methacryloxydiethoxyphenyl)-propan, 2,2'-Bis-(4-acryloxydiethoxyphenyl)-propan, 2,2'-Bis(3(4-phenoxy)-2-hydroxypropan-1-methacrylat)-propan, 2,2'-Bis(3(4-phenoxy)-2-hydroxypropan-1 -acrylat)-propan etc.

Wie vorstehend ausgeführt, kann eine Vielzahl von Methacrylaten und Acrylaten verwendet werden. Diese können allein oder in Kombination aus zwei oder mehreren verwendet werden. Es ist darauf hinzuweisen, daß die vorliegende Erfindung nicht notwendigerweise auf derartige Methacrylate und Acrylate beschränkt ist, vielmehr können in gleicher Weise analoge Verbindungen eingesetzt werden. Andererseits kann die polymerisierbare ungesättigte organische Verbindung, die wenigstens eine CH₂=C(R10)-COO-Gruppe enthält, worin R10 für H oder Methyl steht, in Kombination mit einer polymerisierbaren organischen Verbindung, wie Styrol, N-Vinylpyrrolidon und Divinylbenzol, verwendet werden.

Die verschiedenen Ester von Acrylsäure und Methacrylsäure müssen nicht notwendigerweise allein eingesetzt werden; sie können auch in Kombinationen aus zwei oder mehreren verwendet werden. Insbesondere können Urethan-, Epoxy- und Polyol(meth)acrylate 50% oder mehr des Gesamtgewichts der Komponente (B) ausmachen und zwar der polymerisierbaren ungesättigten organischen Verbindung, die wenigstens eine CH₂=C(R10)-COO-Gruppe enthält, worin R10 für H oder Methyl steht. Urethan(meth)acrylat ist ein allgemeiner Begriff, der auf Acrylate bzw. Methacrylate mit einem Urethanskelett angewandt wird und umfaßt beispielsweise Carbamatverbindungen, wie sie vorstehend erwähnt wurden. Das Polyol(meth)acrylat definiert einen Ester eines 2- oder mehrwertigen Alkohols mit Acryl bzw. Methacrylsäure. Epoxy(meth)acrylat ist ein allgemeiner Begriff, der auf Acryl- bzw. Methacrylatester angewandt wird, die erhalten werden durch Reaktion von Epoxyverbindungen mit Acrylat- oder Methacrylatestern.

Es können auch monomere und polymere Acrylate und Methacrylate verwendet werden. Vorteilhaft eingesetzt werden können auch langkettige Monomere, beispielsweise die aus der US-A-3 066 112 bekannten Monomere auf der Basis von Bisphenol-A und Glycidylmethacrylat oder deren durch Addition von Isocyanaten entstandenen Derivate.

Geeignet sind auch Verbindungen des Typs Bisphenol-A-diethyloxy(meth)acrylat und Bisphenol-A-dipropyloxy(meth)acrylat. Weiterhin Verwendung finden können die oligo-ethoxylierten und oligo-propoxylierten Bisphenol-A-diacryl- und -dimethacrylsäureester. Gut geeignet sind weiter die Acrylsäure- und Methacrylsäureester mindestens bifunktioneller aliphatischer Alkohole, beispielsweise Triethylenglykol-di(meth)-acrylat, Ethylenglykol-di(meth)-acrylat, Hexandiol-di(meth)-acrylat und Trimethylpropan-tri(meth)-acrylat. Besonders geeignet sind auch die in der DE-C-2 816 823 genannten Diacryl- und Dimethacrylsäureester des Bis(hydroxy-methyl)-tricyclo[5.2.1.0^{2,6}]-decans und die Diacryl- und Dimethacrylsäureester der mit 1 bis 3 Ethylenoxid- und/oder Propylenoxidenheiten verlängerten Verbindungen des Bis(hydroxymethyl)-tricyclo[5.2.1.0^{2,6}]-decans.

Gut geeignete Monomere sind auch die in der EP-A-0 235 826 beschriebenen Methacrylsäureester, z.B. Triglykolsäure-bis[3(4)-methacryloxymethyl-8(9)-tricyclo[5.2.1.0^{2,6}]-decylmethylester.

Selbstverständlich können auch Gemische aus Monomeren und/oder aus hieraus hergestellten ungesättigten Polymeren verwendet werden.

Der Bestandteil (B) in einer Menge von 0 bis 98,99 Gew.-%, vorzugsweise 5 bis 70 Gew.-% in den erfindungsgemäßen Mischungen vor.

Als Initiatoren und Aktivatoren des Bestandteiles (C) eignen sich Initiatorsysteme, die die radikalische Polymerisation der Monomeren bewirken, z.B. Photoinitiatoren oder sogenannte Redoxsysteme.

Als Photoinitiatoren eignen sich beispielsweise α-Diketone, wie Campherchinon, in Verbindung mit sekundären und tertiären Aminen, oder Mono- und Bisacylphosphinoxide, wie 2,4,6-Trimethylbenzoyldiphenyl-phosphinoxid und Bis-(2,6-dichlorbenzoyl)-4-n-propylphenyl-phosphinoxid. Es eignen sich aber auch andere Verbindungen dieses Typs, wie sie beispielsweise in den europäischen Patentveröffentlichungsschriften EP-A-0 073 413, EP-A-0 007 508, EP-A-0 047 902, EP-A-0 057 474 und EP-A-0 184 095 beschrieben sind.

Die Konzentration der Photoinitiatoren beträgt in bevorzugter Weise 0,01 bis 3 Gew.-% und insbesondere 0,1 bis 2 Gew.-% der Zubereitung.

Als Redoxinitiatorsysteme eignen sich beispielsweise Peroxidverbindungen zusammen mit sogenannten Aktivatoren. Als organische Peroxidverbindungen kommen dabei insbesondere Verbindungen wie Lauroylperoxid, Benzoylperoxid, o-sowie p-Chlorbenzoylperoxid und p-Methylbenzoylperoxid in Betracht.

Als Aktivatoren eignen sich beispielsweise tertiäre aromatische Amine, wie die aus der US-A-3 541 068 bekannten N,N-Bis-(hydroxyalkyl)-3,5-xylidine sowie die aus der DE-A-2 658 530 bekannten N,N-Bis-(hydroxyalkyl)-3,5-di-t-butylaniline sowie N,N-Bis-(hydroxyalkyl)-3,4,5-trimethylaniline. Gut geeignete Aktivatoren sind auch die in der DE-B-1 495 520 beschriebenen Barbitursäuren und Barbitursäurederivate sowie die in der EP-A-0 059 451 beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Dioctyl-4-isobutylmalonylsulfamid. Zur weiteren Beschleunigung wird die Polymerisation hierbei vorzugsweise in Gegenwart von Schwermetallverbindungen und ionogenem Halogen oder Pseudohalogen durchgeführt. Als Schwermetall ist Kupfer, als Halogenid das Chloridion besonders geeignet. Das Schwermetall wird geeigneterweise in Form löslicher organischer Verbindungen eingesetzt. Ebenso werden die Halogenid- und Pseudohalogenidionen geeigneterweise in Form von löslichen Salzen eingesetzt, beispielsweise genannt seien die löslichen Aminhydrochloride sowie quartäre Ammoniumchloridverbindungen.

Geeignete Redoxsysteme sind auch die aus "Redox Polymerisation", G.S. Misra and U.D.N. Bajpaj, Prog. Polym. Sci., 8, 61-131 (1982) bekannten.

Wenn die erfindungsgemäßen polymerisierbaren Zusammensetzungen als (C) ein Redoxinitiatorsystem aus organischem Peroxid und Aktivator enthalten, so sind vorzugsweise Peroxid und Aktivator in räumlich voneinander getrennten Teilen der erfindungsgemäßen Zusammensetzungen vorhanden, die erst unmittelbar vor der Anwendung homogen miteinander vermischt werden. Enthält die erfindungsgemäße Zusammensetzung als Bestandteil (C) nebeneinander organisches Peroxid, Kupferverbindung, Halogenid und Malonylsulfamid, so ist es insbesondere sinnvoll, daß organisches Peroxid, Malonylsulfamid und die Kombination Kupferverbindung/Halogenid in drei räumlich voneinander getrennten Bestandteilen vorliegen. Beispielsweise können organisches Peroxid, polymerisierbare Monomere sowie Füllstoffe zu einer Paste verknetet sein und die anderen Komponenten in oben beschriebener Weise jeweils mit einer geringen Menge an Füllstoffen oder insbesondere Thixotropie-Hilfsmitteln, wie silanisierter Kieselsäure, und einem Weichmacher, beispielsweise Phthalat, zu zwei separaten Pasten verknetet sein. Andererseits können die polymerisierbaren Monomeren auch zusammen mit Kupferverbindung/Halogenid und Füllern vorliegen. Der Bestandteil (E) kann, falls die erfindungsgemäße Zusammensetzung in räumlich voneinander getrennten Bestandteilen vorliegt, in jedem dieser Bestandteile vorhanden sein.

Geeignete Lösungsmittel gemäß Bestandteil (D) können anorganischer oder organischer Natur sein. Selbstverständlich kann auch eine Mischung aus diesen Lösungsmitteln verwendet werden. Bevorzugte Beispiele sind Wasser (anorganisches Lösungsmittel) und Ketone oder Alkohole (organische Lösungsmittel), beispielsweise Ethanol und Aceton oder Mischungen hiervon. Sie sind in den Zusammensetzungen in einer Menge von 0 bis 90 Gew.-%, vorzugsweise 0 bis 50 Gew.-%, bezogen auf das Gesamtgewicht der Zusammensetzung, enthalten.

Als Bestandteil (E) können anorganische und/oder organische Füllstoffe, Pigmente, Farbstoffe, Thixotropie-Hilfsmittel, Weichmacher, Verdünner, Radikalfänger, Stabilisatoren und andere Hilfsstoffe in der Zusammensetzung enthalten sein.

Anorganische Füllstoffe können beispielsweise Quarz, gemahlene Gläser, nicht wasserlösliche Fluoride, wie z.B. CaF₂ oder SrF₂, Kieselgele sowie Kieselsäure, insbesondere pyrogene Kieselsäure oder deren Granulate, sein. Sie sind in einer Konzentration von 0 bis 90 Gew.-%, vorzugsweise von 20 bis 80 Gew.-%, bezogen auf die Gesamtmasse aller Komponenten, in den erfindungsgemäßen Zusammensetzungen enthalten. Zum besseren Einbau in die Polymermatrix kann es von Vorteil sein, die Füllstoffe sowie gegebenenfalls röntgenopake Zusatzstoffe zu hydrophobieren. Übliche Hydrophobierungsmittel sind Silane, beispielsweise Trimethoxymethacryloxypropylsilan. In einer Ausführungsform sind sämtliche eingesetzten anorganischen Füllstoffe silanisiert, vorzugsweise mit Trimethoxymethacryloxypropylsilan. Die Menge des eingesetzten Silans beträgt üblicherweise 0,5 bis 10 Gew.-%, bezogen auf anorganische Füllstoffe, bevorzugt 1 bis 6 %, ganz besonders bevorzugt 2 bis 5 Gew.-%, bezogen auf anorganische Füllstoffe. Die maximale mittlere Korngröße der anorganischen Füllstoffe beträgt vorzugsweise 15 µm, insbesondere 8 µm. Ganz besonders bevorzugt werden Füllstoffe mit einer mittleren Korngröße von unter 3 um eingesetzt.

Als Füllstoffe eignen sich auch bereits fertig pigmentierte Polymethylmethacrylatperlen oder andere pulverisierte organische Polymerisate. Zur Erhöhung der Flexibilität der Massen kann es auch vorteilhaft sein, lösliche organische Polymere einzusetzen. Geeignet sind z.B. Polyvinylacetat sowie die Copolymeren auf Basis Vinylchlorid/Vinylacetat, Vinylchlorid/Vinylisobutylether und Vinylacetat/Maleinsäuredibutylether. Gut geeignet als zusätzliche Weichmacher sind beispielsweise Dibutyl-, Dioctyl- und Dinonylphthalate.

Als Füllstoffe zur Erhöhung der Fluoridabgabe der erfindungsgemäßen Mischungen können die aus der EP-0 717 977 bekannten komplexen Fluoride der allgemeinen Formel AₙMFₘ eingesetzt werden. Es bedeuten hierin A ein mehrwertiges Kation, M ein Metall der III. bis V. Haupt- oder II. bis V. Nebengruppe, n eine ganze Zahl von 1 bis 3 und m eine ganze Zahl von 3 bis 6. Die vorzugsweisen Ausführungsformen der EP-A-0 717 977 gelten hiermit ausdrücklich auch für diese Erfindung.

Radikalfänger und Stabilisatoren werden bevorzugt in Mengen von 50 bis 5000 ppm, besonders bevorzugt in Mengen von 200 bis 1000 ppm eingesetzt. Bevorzugte Verbindungen sind 4-Methoxyphenol, 2-tert.-Butyl-4,6-dimethyl-phenol oder 2,6-ditert.-Butyl-4-methyl-phenol.

Die Erfindung soll nachfolgend durch Beispiele näher erläutert werden. Jedoch ist die Erfindung keinesfalls auf diese Beispiele beschränkt.

### Herstellungsbeispiel 1

### Herstellung eines Vertreters von (1)

50 g Maleinsäureanhydrid werden in 290 g 1,2-Xylol gelöst und auf Siedetemperatur erhitzt. Man dosiert 3 g Di-tert.-butylperoxid zu und rührt noch 2,5 Stunden. Nach Erkalten des Ansatzes setzt sich das Produkt als gelbe Masse ab und wird durch Dekantieren isoliert.

### Herstellungsbeispiel 2

### Herstellung eines Vertreters von(1)

20 g Maleinsäureanhydrid werden in 116 g 1,4-Xylol auf Siedetemperatur erhitzt und mit 1,2 g Di-tert.-butylperoxid versetzt. Der Ansatz wird 6 Stunden auf Rückfluß gehalten und anschließend das überschüssige Lösungsmittel abgedampft. Das Produkt fällt als gelbe, zähe Flüssigkeit an.

### Herstellungsbeispiel 3

### Herstellung eines Vertreters von (1)

20 g Maleinsäureanhydrid werden in 132 g Mesitylen auf Rückfluß erhitzt. Man dosiert 1,2 g Di-tert.-butylperoxid zu und rührt noch 6 Stunden bei Siedetemperatur. Nach Abdampfen des Lösungsmittels verbleibt das Produkt als gelbe, zähe Flüssigkeit.

### Herstellungsbeispiel 4

### Herstellung eines Vertreters von (A1)

Von dem gemäß Herstellungsbeispiel 1 erhaltenen Produkt werden 10 g in 15 ml THF gelöst und mit 100 mg Schwefelsäure versetzt. Der Ansatz wird auf 50°C erhitzt, mit 5 g 2-Hydroxyethylmethacrylat versetzt und zwei Tage bei 50°C gerührt. Danach wird mit Wasser versetzt und stehengelassen. Das Monomer (A1) wird nach Extraktion und anschließendem Abdampfen des Lösungsmittels als gelbes, zähflüssiges Material erhalten.

### Herstellungsbeispiel 5

### Herstellung eines Vertreters von (A1)

Von dem nach Herstellungsbeispiel 1 erhaltenen Produkt werden 8 g in 15 ml THF gelöst und mit 80 mg Schwefelsäure sowie 6,4 mg Jonol versetzt. Nach Erhitzen auf 60°C werden 8,3 g HEMA zugegeben. Der Ansatz wird 4 Tage auf Temperatur gehalten, mit Wasser versetzt und extrahiert. Nach Abdampfen des Lösungsmittels wird das Monomer (A1) als gelbes zähflüssiges Öl erhalten.

### Herstellungsbeispiel 6

### Herstellung eines Vertreters von (A1)

Von dem gemäß Herstellungsbeispiel 1 erhaltenen Produkt werden 15 g in 30 ml Tetrahydrofuran gelöst und mit 0,25 g Natriumacetat versetzt. Der Ansatz wird auf 50°C erwärmt und es werden 7,5 g 2-Hydroxyethylmethacrylat zugegeben. Nach zweitägigem Rühren bei 50°C wird Wasser zugesetzt und einen Tag nachgerührt. Das Monomer (A1) wird nach Extrahieren und Abdampfen des Lösungsmittels als gelbes, zähflüssiges Material erhalten.

### Herstellungbeispiel 7

### Herstellung eines Vertreters von (A2)

24,8 g Tetrahydrofuran-2,3,4,5-tetracarbonsäure, 28,4 g Glycidylmethacrylat (GMA), 100 ml Tetrahydrofuran werden unter Rühren auf 70°C erwärmt. Nach 24 Stunden wird der Ansatz filtriert und unter Lichtausschluß in der Kälte eingeengt. Nach Abdampfen des Lösungsmittels wird ein viskoses, rotbraunes Produkt erhalten.

### Herstellungbeispiel 8

### Herstellung eines Vertreters von (A2)

20 g all-cis-Cyclohexan-1,2,3,4,5,6-hexacarbonsäure, 0,16 Mol GMA, 50 ml THF werden 20 Stunden bei 65°C gerührt. Die Flüssigkeit wird nach Abfiltration des ausgefallenen Feststoffes in der Kälte unter Lichtausschluß eingeengt, wobei ein klares, viskoses Produkt erhalten wird.

### Herstellungbeispiel 9

### Herstellung eines Vertreters von (A2)

0,1 Mol Tetrahydrofuran-2,3,4,5-tetracarbonsäure-dianhydrid, 0,2 Mol 2-Hydroxyethyl-methacrylat, 100 ml THF werden 20 Stunden bei Raumtemperatur gerührt. Die Lösung wird in der Kälte unter Lichtausschluß eingeengt, wonach ein rotbraunes, klares, viskoses Produkt erhalten wird. Das Endprodukt fällt nach Entfernen des Lösungsmittels als wachsartiger Niederschlag an, der 8 Stunden getrocknet werden muß.

### Beispiele 1 bis 4, Vergleichsbeispiele 1 und 2

### Erfindungsgemäße polymerisierbare Haftvermittler

**Tabelle 1:**

| **Bestandteil (Gew.-%)** | **Beispiel 1** | **Beispiel 2** | **Beispiel 3** | **Beispiel 4** | **Vergleichs- beispiel 1** | **Vergleichs- beispiel 2** |
|---|---|---|---|---|---|---|
| **Vertreter von (A1) gemäß Herstellungs- beispiel 4** | 20 | 0 | 15 | 20 | 0 | 0 |
| **Vertreter von (A1) gemäß Herstellungs- beispiel 6** | 0 | 0 | 0 | 20 | 0 | 0 |
| **Vertreter von (A2) gemäß Herstellungs- beispiel 7** | 0 | 20 | 15 | 0 | 0 | 0 |
| **BTDA-HEMA** (*) | 0 | 0 | 0 | 0 | 20 | 0 |
| **HEMA-Phthalat**(**) | 0 | 0 | 0 | 0 | 0 | 20 |
| **HEMA** | 20 | 20 | 30 | 20 | 20 | 20 |
| **TEGDMA** | 19,5 | 19,5 | 19,5 | 14,5 | 19,5 | 19,5 |
| **Bis-GMA** | 20 | 20 | 20 | 15 | 20 | 20 |
| **Campherchinon** | 0,3 | 0,3 | 0,3 | 0,3 | 0,3 | 0,3 |
| **Dimethylaminoethylbenzoat** | 0,2 | 0,2 | 0,2 | 0,2 | 0,2 | 0,2 |
| **Kaliumhexafluorotitanat** | 0 | 0 | 0 | 10 | 0 | 0 |
| **Aceton** | 20 | 0 | 0 | 20 | 20 | 20 |
| **Wasser** | 0 | 20 | 0 | 0 | 0 | 0 |

| | | | | | | |
|---|---|---|---|---|---|---|
| (*): BTDA-HEMA ist ein Umsetzungsprodukt von 3,3'-3,3'-Benzophenontetracarbonsäuredianhydrid mit der 2-fachen Menge an 2-Hydoxyethylmethacrylat (vgl. US-A-4 659 751). | | | | | | |
| (**): HEMA-Phthalat ist das Umsetzungsprodukt von Phthalsäureanhydrid mit 2-Hydroxyethylmethacrylat (E. Mashuhara, K. Kojhima, N. Tarumi, N. Nakabayashi, H. Hotta, Rep. Inst. Med. Dent. Eng. 1, 29 (1967). | | | | | | |

Die Überprüfung des Haftverbundes erfolgte durch einen Haftabzugsversuch an Rinderzähnen. Pro Versuch wurden 5 frisch extrahierte Rinderzähne mittels Schleifpapier soweit abgeschliffen, daß eine ausreichend große freiliegende Dentinfläche entstand. Auf diese Flächen wurden jeweils Wachsplättchen mit einem ausgestanzten Loch von 4 mm geklebt, um eine standardisierte Haftfläche zu erhalten. Zur weiteren Standardisierung wurden alle so erhaltenen Prüfkörper in Anlehnung an das praxisübliche Vorgehen ("all-etch Technik") mittels einer üblichen Phosphorsäurelösung (Ätzgel Minitip, Fa. ESPE Dental-Medizin, Seefeld) für 15 Sekunden angeätzt. Auf die derart vorbereiteten Dentinflächen wurden die gemäß den Beispielen 1 bis 4 und Vergleichsbeispielen 1 und 2 zubereiteten Haftvermittler aufgetragen und mittels eines Lichtpolymerisationsgerätes (Elipar, Fa. ESPE) polymerisiert. Anschließend wurde ein Dentalcomposite (Pertac, Fa. ESPE) in die Aussparungen der Wachsplättchen eingebracht und auspolymerisiert. Nach 24-stündiger Lagerung bei 36°C und 100% Luftfeuchtigkeit wurden die Wachsplättchen entfernt und der Compositeprüfkörper in einem Zugversuch (Zwick Universalprüfmaschine) abgezogen.

Die folgende Tabelle (2) stellt die Mittelwerte der erhaltenen Haftfestigkeiten in MPa zusammen.

Zum Vergleich mit Haftvermittlern aus dem Stand der Technik enthält Tabelle (2) ebenfalls gemäß vorheriger Versuchsbeschreibung mit diesen Haftvermittlern erhaltene Haftwerte, wobei anstelle des Composites Pertac dabei das jeweils vom Hersteller empfohlene Composite verwendet wurde.

**Tabelle 2:**

| **Haftvermittler** | **Haftfestigkeit in MPa** |
|---|---|
| Beispiel 1 | 6,0 |
| Beispiel 2 | 6,1 |
| Beispiel 3 | 8,9 |
| Beispiel 4 | 7,8 |
| Vergleichsbeispiel 1 | 2,6 |
| Vergleichsbeispiel 2 | 0,8 |
| Syntac SC, Fa. Vivadent | 2,1 |
| Prime & Bond, Fa. Dentsply | 3,8 |

Diese Tabelle zeigt deutlich die Überlegenheit der erfindungsgemäßen Zusammensetzungen sowohl gegenüber dem Einsatz bekannter Haftmoleküle (Vergleichsbeispiele 1 und 2) als auch gegenüber dem Einsatz kommerzielle verfügbarer Adhäsivsysteme (Syntac SC bzw. Prime & Bond).

Die angegebenen Werte für die Haftung sind als Relativwerte und nicht als Absolutwerte anzusehen, da bei einem Vergleich der Ergebnisse mehrerer Testreihen der identischen Experimente die Relationen zwar gleich sind, die ermittelten Werte aber im Vergleich der Testreihen untereinander voneinander abweichen können. Aufgrund der streuenden Qualität der verwendeten extrahierten Zähne werden in manchen Fällen mehr kohäsive Brüche beobachtet.

## Patentansprüche

1. Polymerisierbare, vernetzbare Zusammensetzung, **dadurch gekennzeichnet, daß** sie
(A) 1 - 99,99 gew.-% mindestens eines Umsetzungsproduktes von OH-funktionellen (Meth)acrylaten mit
(i) den Verbindungen der Formel (1): 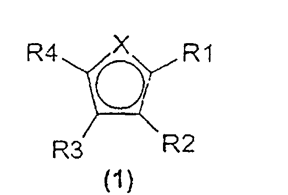 in welcher:
R1, R2, R3 und R4 unabhängig voneinander ausgewählt sind aus der Gruppe:
(a) Wasserstoff,
(b) unverzweigte oder verzweigte Kohlenwasserstoffreste mit 1 bis 20 C-Atomen,
(c) unverzweigte oder verzweigte Heteroaliphaten mit 1 bis 20 C-Atomen und Heteroatomen ausgewählt aus der Gruppe N, O, S,
(d) Halogene und Pseudohalogene, bevorzugt F, Cl, Br, CN, SCN, und
(e) dem Fragment der Formel (2): 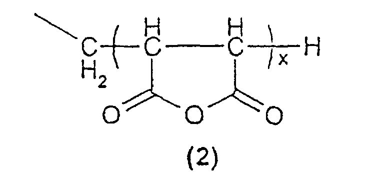 in welcher x ganze Zahlenwerte zwischen 1 und 8 annehmen kann,
und X ausgewählt ist aus der Gruppe O, S, 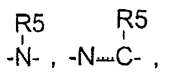 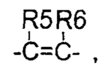 mit R5 bzw. R6 entsprechend der obigen Definition von R1 bis R4,
mit der Maßgabe, daß mindestens zwei beliebige Vertreter der Gruppe R1 bis R4, R5, R6 dem Fragment der Formel (2) entsprechen, wobei das Substitutionsmuster der Fragmente der Formel (2) ortho, meta oder para sein kann,
und/oder mit
(ii) mindestens einer ringförmigen Polycarbonsäure mit mindestens 4 Ring-Kohlenstoffatomen und/oder deren Anhydriden mit gesättigtem Ringsystem und mind. vier Carboxylgruppen sowie 0 oder 1 Hetero-Ringatomen aus der Gruppe N, O, S der allgemeinen Formel (3): 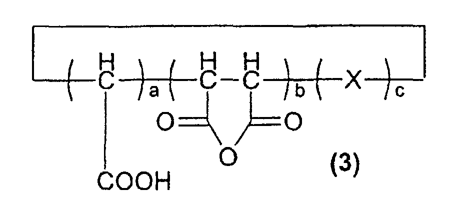
worin a = 0 bis 6,
b = 0 bis 3,
c = 0 oder 1, und
X = O, NR1 oder S bedeuten,
wobei R1 die obige Bedeutung hat, ausgenommen (e) ein Fragment der Formel (2), und
wobei mindestens eine Carboxylgruppe und/oder Anhydridgruppe der vorstehend bezeichneten Substanzgruppen mit ungesättigten polymerisierbaren Gruppen umgesetzt ist
(B) 0 bis 98,99 Gew.-% einer oder mehreren polymerisierbaren; ungesättigten organischen Verbindungen mit wenigstens einer CH₂=C(R10)-COO-Gruppe, worin R10 = H oder Methyl ist
(C) 0,01 bis 5 Gew.-% initiatoren und ggf. Aktivatoren
(D) 0 bis 90 Gew.-% übliche Lösungsmittel
(E) 0 bis 90 Gew.-% übliche Füllstoffe, Pigmente, Thixotropiehilfsmittel, Weichmacher, Verdünner, starrmachende Monomere, Radikalfänger, Stabilisatoren, sonstige Hilfsmittel
enthält, wobei sich die Angabe der Gew.-% jeweils auf die Summe aller Bestandteile (A) bis (E) bezieht.

2. Polymerisierbare, vernetzbare Zusammensetzung nach Anspruch 1, **dadurch gekennzeichnet, daß** sie als Bestandteil (A) ein Umsetzungsprodukt von OH-funktionellen (Meth)acrylaten mit Verbindungen der Formel (4) enthält: 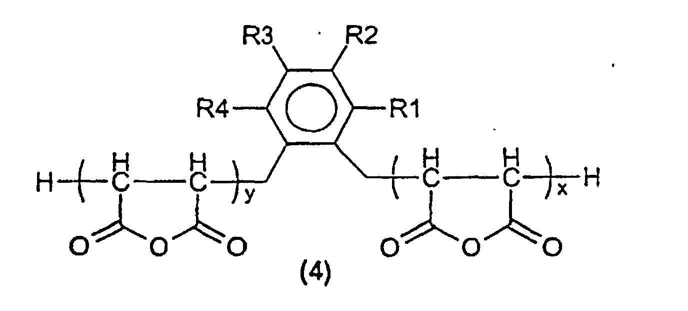 in welcher x und y unabhängig voneinander ganze Zahlenwerte zwischen 1 und 8 annehmen können und R1, R2, R3 und R4 die in Anspruch 1 angegebenen Bedeutungen (a) bis (d) haben.

3. Polymerisierbare, vernetzbare Zusammensetzung nach Anspruch 1, **dadurch gekennzeichnet, daß** sie als Bestandteil (A) das Umsetzungsprodukt von Verbindungen der Formel (1), die durch Reaktion von 1,2-Xylol mit Maleinsäureanhydrid entstanden sind, mit OH-funktionellen (Meth)acrylaten enthält.

4. Polymerisierbare, vernetzbare Zusammensetzung nach Anspruch 1, **dadurch gekennzeichnet, daß** sie als Bestandteil (A) das Umsetzungsprodukt von Verbindungen der Formel (1), die durch Reaktion von 1,4-Xylol mit Maleinsäureanhydrid entstanden sind, mit OH-funktionellen (Meth)acrylaten enthält.

5. Polymerisierbare, vernetzbare Zusammensetzung nach Anspruch 1, **dadurch gekennzeichnet, daß** sie als Bestandteil (A) das Umsetzungsprodukt von Verbindungen der Formel (1), die durch Reaktion von Mesitylen mit Maleinsäureanhydrid entstanden sind, mit OH-funktionellen (Meth)acrylaten enthält.

6. Polymerisierbare, vernetzbare Zusammensetzung nach Anspruch 1, **dadurch gekennzeichnet, daß** sie als Bestandteil (A) Verbindungen enthalten, die durch Umsetzung von Tetrahydrofuran-2,3,4,5-tetracarbonsäure mit OH-funktionellen (Meth)acrylaten entstanden sind.

7. Polymerisierbare, vernetzbare Zusammensetzung nach Anspruch 1, **dadurch gekennzeichnet, daß** sie als Bestandteil (A) Verbindungen enthalten, die durch Umsetzung von Tetrahydrofuran-2,3,4,5-tetracarbonsäure-dianhydrid mit OH-funktionellen (Meth)acrylaten entstanden sind.

8. Polymerisierbare, vernetzbare Zusammensetzung nach Anspruch 1, **dadurch gekennzeichnet, daß** sie als Bestandteil (A) Verbindungen enthalten, die durch Umsetzung von All-cis-cyclohexanhexacarbonsäure mit OH-funktionellen (Meth)acrylaten entstanden sind.

9. Polymerisierbare, vernetzbare Zusammensetzung nach einem der Ansprüche 3 bis 8, **dadurch gekennzeichnet, daß** sie als Bestandteil (A) das Umsetzungsprodukt mit Isopropyliden-bis-[2-hydroxy-3-(4-phenoxy)-propyl]methacrylat oder 2-Hydroxyethylmethacrylat als OH-funktionelles (Meth)acrylat enthält.

10. Verwendung von polymerisierbaren, vernetzbaren Zusammensetzungen nach einem der Ansprüche 1 bis 9 zum Verkleben, Vergießen oder Beschichten von Substraten.

11. Verwendung von polymerisierbaren, vernetzbaren Zusammensetzungen nach einem der Ansprüche 1 bis 9 auf dem zahnmedizinischen oder zahntechnischen Sektor, als dentaler Haftvermittler, dentales Haftfüllungsmaterial, dentaler Haftzement oder dentaler Sealer.

## Claims

1. Polymerizable, cross-linkable composition, **characterized in that** it contains
(A) 1-99.99 wt.-% of at least one reaction product of OH-functional (meth)acrylates with
(i) the compounds of formula (1): 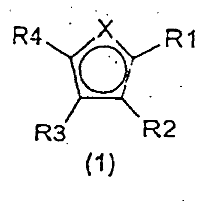 in which:
R1, R2, R3 and R4 are selected independently from each other from the group:
(a) hydrogen,
(b) linear or branched hydrocarbon radicals with 1-20 C atoms,
(c) linear or branched heteroaliphatics with 1 to 20 C atoms and heteroatoms selected from the group N, O, S,
(d) halogens and pseudo halogens, preferably F, Cl, Br, CN, SCN, and
(e) the fragment of formula (2): 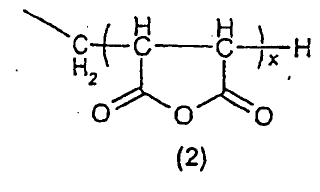 in which x can assume whole numerical values between 1 and 8, and X is selected from the group O, S, 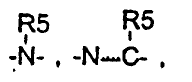 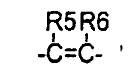 with R5 or R6 corresponding to the above definition of R1 to R4,
on the condition that at least two random representatives of the group R1 to R4, R5, R6 correspond to the fragment of formula (2), the substitution pattern of the fragments of formula (2) being able to be ortho, meta or para,
and/or with
(ii) at least one ring-shaped polycarboxylic acid with at least 4 ring carbon atoms and/or their anhydrides with a saturated ring system and at least four carboxyl groups as well as 0 or 1 hetero ring atoms from the group N, O, S of the general formula (3): 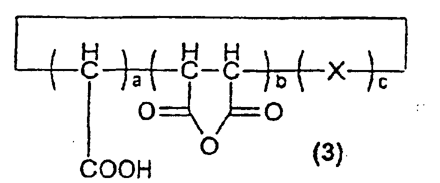
where a = 0 to 6,
b = 0 or 3,
c = 0 or 1, and
X = 0, NR1 or S,
R1 having the above meaning, except for (e) a fragment of the formula (2), and
at least one carboxyl group and/or anhydride group of the above-named substance groups being reacted with unsaturated polymerizable groups
(B) 0 to 98.99 weight-% of one or more polymerizable, unsaturated organic compounds with at least one CH₂=C(R10)-COO group, where R10 = H or is methyl
(C) 0.01 to 5 weight-% initiators and optionally activators
(D) 0 to 90 weight-% customary solvents
(E) 0 to 90 weight-% of conventional fillers, pigments, thixotropy auxiliary agents, plasticizers, diluting agents, rigidifying monomers, radical captors, stabilizers, other auxiliaries,
the given weight-% referring in each case to the sum of all the components (A) to (E)

2. Polymerizable, cross-linkable composition according to claim 1, **characterized in that** it contains as component (A) a reaction product of OH-functional (meth)acrylates with compounds of formula (4): 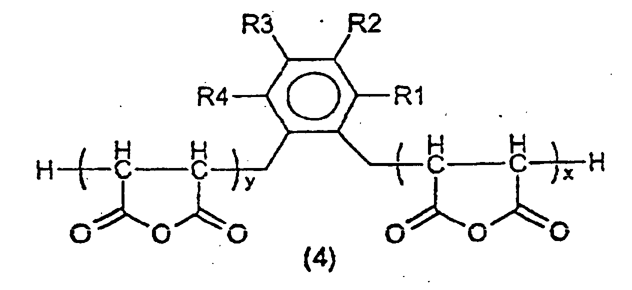 in which x and y, independently of each other, can assume whole numerical values between 1 and 8, and R1, R2, R3 and R4 have the meanings (a) to (d) specified in claim 1.

3. Polymerizable, cross-linkable composition according to claim 1, **characterized in that** it contains as component (A) the reaction product of compounds of formula (1), which have formed by the reaction of 1,2-xylene with maleic anhydride, with OH-functional (meth)acrylates.

4. Polymerizable, cross-linkable composition according to claim 1, **characterized in that** it contains as component (A) the reaction product of compounds of formula (1), which have formed by the reaction of 1,4-xylene with maleic anhydride, with OH-functional (meth)acrylates.

5. Polymerizable, cross-linkable composition according to claim 1, **characterized in that** it contains as component (A) the reaction product of compounds of formula (1), which have formed by the reaction of mesitylene with maleic anhydride, with OH-functional (meth)acrylates.

6. Polymerizable, cross-linkable composition according to claim 1, **characterized in that** it contains as component (A) compounds which have formed by the reaction of tetrahydrofuran-2,3,4,5-tetracarboxylic acid with OH-functional (meth)acrylates.

7. Polymerizable, cross-linkable composition according to claim 1, **characterized in that** it contains as component (A) compounds, which have formed by the reaction of tetrahydrofuran-2,3,4,5-tetracarboxylicdianhydride with OH-functional (meth)acrylates.

8. Polymerizable, cross-linkable composition according to claim 1, **characterized in that** it contains as component (A) compounds, which have formed by the reaction of all-cis-cyclohexane hexacarboxylic acid with OH-functional (meth)acrylates.

9. Polymerizable, cross-linkable composition according to one of claims 3 to 8, **characterized in that** it contains as component (A) the reaction product with isopropylidene-bis-[2-hydroxy-3-(4-phenoxy)-propyl]methacrylate or 2-hydroxyethyl methacrylate as OH-functional (meth)acrylate.

10. Use of polymerizable, cross-linkable compositions according to one of claims 1 to 9 for gluing, casting or coating substrates.

11. Use of polymerizable, cross-linkable compositions according to one of claims 1 to 9 in the fields of dentistry or dental technology as a dental adhesion promoter, dental adhesion filling material, dental adhesion cement or dental sealer.

## Revendications

1. Composition polymérisable, réticulable, **caractérisée en ce qu'**elle contient :
(A) 1 à 99,99% en poids d'au moins un produit de réaction de (méth)acrylates à fonction OH, avec
(i) les composés de la formule (1) : 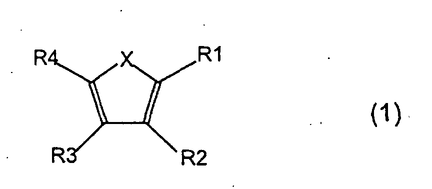 dans laquelle :
R1, R2, R3 et R4 sont choisis indépendamment l'un de l'autre parmi le groupe consistant en :
(a) l'atome d'hydrogène ;
(b) un reste hydrocarbure linéaire ou ramifié, avec 1 à 20 atomes C ;
(c) un radical hétéroaliphatique linéaire ou ramifié, avec 1 à 20 atomes C et des hétéroatomes choisis parmi les atomes N, O, S ;
(d) un atome d'halogène ou pseudohalogène, de préférence F, Cl, Br, CN, SCN, et
(e) le fragment de la formule (2) : 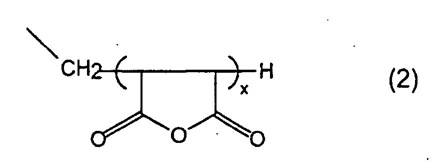
dans laquelle x peut prendre une valeur numérique entière allant de 1 à 8, et
X est choisi parmi le groupe O, S, -N(R5)-, -N-C(R5)-, -C(R5)=C(R6)-, avec R5 ou R6 correspondant à la définition de R1 à R4 ci-dessus,
avec la condition qu'au moins deux représentants quelconques du groupe R1 à R4, R5, R6 correspondent au fragment de la formule (2), où le type de substitution des fragments de la formule (2) peut être ortho, méta
ou para,
et/ou avec
(ii) au moins un poly(acide carboxylique) cyclique avec au moins 4 atomes de carbone cycliques et/ou leur anhydride avec un système cyclique saturé et au moins quatre radicaux carboxyle ainsi que 0 ou 1 hétéroatome cyclique du groupe N, O, S, de la formule générale (3) : 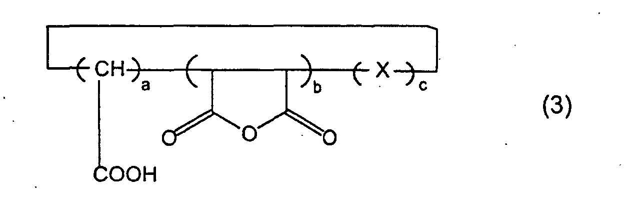 où a = 0 à 6 ;
b = 0 à 3 ;
c = 0 ou 1, et
X = O, NR1 ou S,
où R1 a la signification ci-dessus, à l'exception de (e) un fragment de la formule (2), et
où au moins un radical carboxyle et/ou radical anhydride du groupe de substances caractérisé précédemment a réagi avec des radicaux polymérisables insaturés ;
(B) 0 à 98,99% en poids d'un ou de plusieurs composés organiques polymérisables insaturés avec au moins un radical CH₂=C(R10)-COO-, où R10 = H ou méthyle ;
(C) 0,01 à 5% en poids d'initiateurs et le cas échéant, d'activateurs ;
(D) 0 à 90% en poids d'un solvant usuel ;
(E) 0 à 90% en poids des charges, pigments, auxiliaires de thixotropie, plastifiants, diluants, monomères rendant rigide, pièges de radicaux, stabilisants, autres auxiliaires usuels,
où les données en % en poids reposent chaque fois, sur la somme de tous les constituants (A) à (E).

2. Composition polymérisable, réticulable selon la revendication 1, **caractérisée en ce qu'**elle contient comme constituant (A), un produit de réaction de (méth)acrylates à fonction OH avec des composés de la formule (4) : 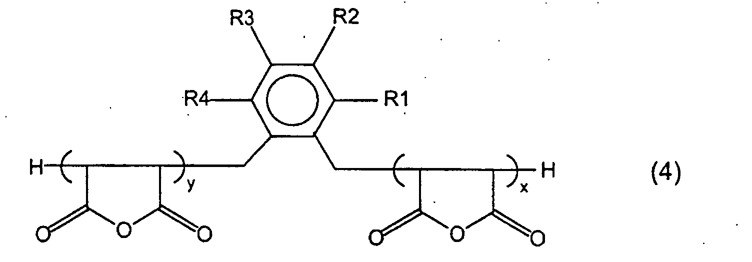 dans laquelle x et y peuvent prendre, indépendamment l'un de l'autre, une valeur numérique entière allant de 1 à 8, et R1, R2, R3 et R4 ont les significations (a) à (d) indiquées à la revendication 1.

3. Composition polymérisable, réticulable selon la revendication 1, **caractérisée en ce qu'**elle contient comme constituant (A), le produit de réaction de composés de la formule (1), qui sont obtenus par réaction de 1,2-xylène avec l'anhydride maléfique, avec des (méth)acrylates à fonction OH.

4. Composition polymérisable, réticulable selon la revendication 1, **caractérisée en ce qu'**elle contient comme constituant (A), le produit de réaction de composés de la formule (1), qui sont obtenus par réaction de 1,4-xylène avec l'anhydride maléique, avec des (méth)acrylates à fonction OH.

5. Composition polymérisable, réticulable selon la revendication 1, **caractérisée en ce qu'**elle contient comme constituant (A), le produit de réaction de composés de la formule (1), qui sont obtenus par réaction du mésitylène avec l'anhydride maléique, avec des (méth)acrylates à fonction OH.

6. Composition polymérisable, réticulable selon la revendication 1, **caractérisée en ce qu'**elle contient comme constituant (A), les composés qui sont formés par la réaction de l'acide tétrahydrofurann-2,3,4,5-tétracarboxylique avec des (méth)acrylates à fonction OH.

7. Composition polymérisable, réticulable selon la revendication 1, **caractérisée en ce qu'**elle contient comme constituant (A), les composés qui sont formés par la réaction du dianhydride de l'acide tétrahydrofurann-2,3,4,5-tétracarboxylique avec des (méth)acrylates à fonction OH.

8. Composition polymérisable, réticulable selon la revendication 1, **caractérisée en ce qu'**elle contient comme constituant (A), les composés qui sont formés par la réaction de l'acide tous-cis-cyclohexanehexacarboxylique avec des (méth)acrylates à fonction OH.

9. Composition polymérisable, réticulable selon l'une quelconque des revendications 3 à 8, **caractérisée en ce qu'**elle contient comme constituant (A), le produit de réaction avec le méthacrylate d'isopropylidène-bis[2-hydroxy-3-(4-phénoxy)propyle] ou le méthacrylate de 2-hydroxyéthyle comme (méth)acrylates à fonction OH.

10. Utilisation de compositions polymérisables, réticulables selon l'une quelconque des revendications 1 à 9, pour l'encollage, la coulée ou l'enduction de substrats.

11. Utilisation de compositions polymérisables, réticulables selon l'une quelconque des revendications 1 à 9, dans le secteur de la médecine dentaire ou de la technique dentaire, comme agent adhésif dentaire, matériau de charge adhésif dentaire, ciment adhésif dentaire ou agent de scellement dentaire.
